# EUROPEAN PATENT APPLICATION

(11) **EP 1 969 938 A2**
(43) Date of publication of application: **17.09.2008**
(21) Application number: 08003162.8
(22) Date of filing: 21.02.2008
(51) Int. Cl.: A01N 59/00, A01N 37/36, A01P 1/00

(54) **Stabilized chlorine dioxide and hypochlorous acid in a liquid biocide**

(30) Priority: 16.03.2007 US 687007
(71) Applicant: Bou-Matic Technologies Corporation, Madison, WI 53716 (US)
(72) Inventor: Stevenson, Randal D., Cottage Grove, WI 53527 (US)
(74) Representative: Gesthuysen, von Rohr & Eggert

(57) **Abstract**

A liquid biocide containing stabilized chlorine dioxide and hypochlorous acid for various uses, including use as a teat dip or dairy animals. The stable, prophylactic biocide consists essentially of 0.10-20.0% by weight of a chlorite salt, 0.1-3.0% by weight of an acid, 0.10-2.0% by weight of an alkaline agent, and the balance water. Chlorite salt reacts with the acid to form germicidal chlorine dioxide and hypochlorous acid. The acid constituency and the alkaline agent constituency regulate the pH of the solution such that the biocide is stable when mixed for a period greater than two months. The present disclosure also includes a method of producing a stable, prophylactic biocide for dairy cattle, the method comprising 0.10-20.0% by weight of a chlorite salt, 0.10-3.0% by weight of an acid, 0.10-2.0% by weight of an alkaline agent, and the balance water in a container, and sealing the container. In the method, the acid and alkaline agent constituencies regulate the pH of the solution such that the formation of hydronium ions from the acid is correspondingly regulated to stabilize the solution for a period greater than two months from the date of sealing.

## Description

This application relates to a stable, prophylactic biocide primarily for use in routine dairy procedures, and to a method of producing such a dioxide.

Antibiotics are used in dairy cattle production primarily to treat or prevent disease and, to a lesser extent, to increase milk production or improve feed efficiency. Thus, antibiotic use in dairy production can be classified as therapeutic, prophylactic or sub-therapeutic. Commonly, prophylactic biocides are used as a teat dip to prevent proliferation of pathogenic microorganisms among dairy animals.

Prior to this invention, chlorine dioxide and hypochlorous acid-based prophylactic biocides were provided in a two-part (binary) system. In such systems, the user is responsible for mixing the two parts on-farm and using the mixture before its efficacy has dissipated. Commonly, these binary systems are acid-chlorite systems that produce germicidal chlorine dioxide and hypochlorous acid. Commonly, lactic acid and/or citric acid are used as the acid part to the binary system and sodium chlorite is used as the alkaline part. A well known binary acid chloride system is the Gladiator^{™} system available from Bou-Matic, LLC, Madison, Wisconsin, USA.

The major drawbacks to such binary systems arise from the fact that chlorine dioxide and hypochlorous acid are inherently instable in solution. Generally, hypochlorous acid quickly forms chlorine dioxide (a gas dissolved in solution) which dissipates. Also, chlorate salts and other non-desirable reactions can take place diminishing the yield of conventional acid-chlorite binary systems. Conventional, binary systems of chlorine dioxide and hypochlorous acid are stable for one week or less. This instability is partially due to the fact that chlorite ions form readily from hypochlorous acid.

Accordingly, there is a need for a liquid biocide using hypochlorous acid and chlorine dioxide as the primary biocidical agents, but that will have greater stability so that a binary system is unnecessary.

The inventor has found that by regulating the amount of acid and modifying the alkaline constituency in a solution of sodium chlorite and water results in a liquid biocide containing chlorine dioxide and hypochlorous acid that is stable for at least two months.

The stable prophylactic biocide of the invention, which is primarily applicable for dairy cattle, consists essentially of 0.10-20.00% by weight of a chlorite salt, 0.10-3.00% by weight of an acid, 0.10-2.00% by weight of an alkaline agent, and the balance water. The acid constituency may be selected from any acid, and, in one embodiment, is selected from the group consisting of citric acid, phosphoric acid, lactic acid, acetylsalicylic acid, and acetic acid or a mixture thereof. The chlorite salt constituency may be selected from any chlorite salt and, in one embodiment, is selected from the group consisting of: sodium chlorite, magnesium chlorite, potassium chlorite and calcium chlorite, or a mixture thereof. The alkaline agent may be selected from any alkaline agent and, in one embodiment, is selected from the group consisting of: sodium hydroxide, potassium hydroxide, calcium hydroxide, sodium carbonate, potassium carbonate, and calcium carbonate or a mixture thereof. The biocide may further include urea, dyes, perfumes, emollients, non-ionic surfactants or rheology modifiers to achieve increased stability and other characteristics sought by consumers.

In a specific embodiment of the prophylactic biocide of the present invention, the biocide consists essentially of 0.10-20.0% by weight sodium chlorite, 0.10-3.00% by weight lactic and/or citric acid, 0.010-2.00% by weight urea, 0.10-2.00% by weight sodium hydroxide, and the balance water. Again, in this embodiment, the biocide may further include dyes, perfumes, emollients, non-ionic surfactants, and/or rheology modifiers to achieve characteristics sought by consumers.

The liquid biocide creates very powerful, broad spectrum germicides, hypochlorous acid and chlorine dioxide. These germicides have proven efficacy against *streptococcus agalactiae, staphylococcus aureus,* and other pathogenic microorganisms. Specifically, the chlorite salt reacts with the acid to form the germicidal chlorine dioxide and hypocholorus acid. More specifically, a hydronium ion (H₃O⁺) reacts with the chlorite salt to form chlorine dioxide and hypochlorous acid. In the liquid biocide of the present application, the acid constituency and the alkaline agent constituency are both supplied in an amount effective to regulate the pH of the solution to provide the aforementioned stability of the biocide. By regulating the pH of the solution, the supply of hydronium ions from the acid is limited such that the chlorite salt reaction with the hydronium ions to form germicidal chlorine dioxide and hypochlorous acid is correspondingly regulated. By regulating the pH of the solution, any hypochlorous acid that is formed is also stabilized such that the production of chlorate ions from hypochlorous acid is reduced. This increases the shelf life of the mixed biocide considerably, from two weeks to over two months. A ph-value of 6.5 to 6.9 gives good results.

The application also contemplates a method for producing a stable, prophylactic biocide for use with dairy animals, the method comprising combining 0.10-20.00% by weight of a chlorite salt, 0.10-3.00% by weight of an acid, 0.10-2.00% by weight of an alkaline agent, and the balance water in a container, and sealing the container. The container may be any suitable container, as are well known in the art. The acid and alkaline agent constituencies regulate the pH of the solution such that the formation of hydronium ions from the acid is correspondingly regulated to stabilize the solution for a period greater than two months from the date of sealing. As in the stable prophylactic biocide, the method for producing this stable, prophylactic biocide involves using any acid, chlorite salt and alkaline agent. Particular embodiments of each of these constituencies are described herein.

In the method, the formulation of chlorine dioxide and hypochlorous acid is regulated by the formation of the hydronium ions from the acid. Furthermore, the regulation of the pH of the solution reduces the formation of chlorate ions derived from hypochlorous acid. This regulation stabilizes the combination created by the method.

Accordingly, a biocide and a method of producing such a biocide are herein disclosed. The biocide may be used as a dairy cow, goat, or sheep teat disinfectant. However, the biocide may be useful in many other instances involving the care of livestock and particularly dairy cows.

### DETAILED DESCRIPTION OF EMBODIMENTS

The stable, prophylactic biocide of the present disclosure is a solution consisting essentially of 0.10-20.0% by weight of a chlorite salt, 0.10-3.0% by weight of an acid, 0.10-2.0% by weight of an alkaline agent, and the balance water.

The acid used in the solution may be any acid. In one embodiment, the acid is selected from the group: citric acid, phosphoric acid, lactic acid, acetic acid, acetylsalicylic acid, or a mixture thereof. In another embodiment, the acid is a mixture of citric acid and lactic acid. In yet another embodiment, the solution includes only lactic acid.

The chlorite salt of the solution may be any chlorite salt. In one preferred embodiment, the chlorite salt is selected from the group: sodium chlorite, magnesium chlorite, potassium chlorite, calcium chlorite, or a mixture thereof. In another embodiment, the chlorite salt is sodium chlorite.

The alkaline agent of the stable, prophylactic biocide may include any source of alkalinity. In one embodiment, the alkaline agent is selected from the group: sodium hydroxide, potassium hydroxide, calcium hydroxide, sodium carbonate, potassium carbonate or a mixture thereof

The stable, prophylactic biocide may also include up to 0.05% of a dye. The dye may be any dye that is stable in chlorine dioxide. In one embodiment, the solution includes FD&C Blue #1. In another embodiment, the dye includes Hostafine Blue from Clariant Corporation of Charlotte, North Carolina. The solution may also include a perfume to add a desired scent to the solution.

In one embodiment, the solution includes up to 2.00% urea to enhance stability of the chlorine dioxide and promote wound healing. In another embodiment, the stable, prophylactic biocide includes up to 20.00% of a non-ionic surfactant. The non-ionic surfactant may be any chlorite-stable surfactant. In one embodiment, the non-ionic surfactant was selected from the group: linear alcohol ethoxylate, nonylphenol ethoxylate, sodium xylene sulfates and quaternary ammonium chlorites. However, as will be recognized by those of ordinary skill in the art, any surfactant stable in solution is acceptable. The solution may also include up to 65% of an emollient. In one embodiment, the emollient is selected from the group: glycerin, sorbitol, propylene glycol, petrolatum, mineral oil, olive oil, coconut oil, lanoline and cocoa butter. However, as will be recognized by those of ordinary skill in the art, any emollient stable in solution is acceptable.

In yet another embodiment, the stable, prophylactic biocide of the present application includes up to 0.65% of a rheology modifier to achieve the preferred viscosity. The rheology modifier may be selected from the group: xanthan gum, carboxymethylcellulose, microcrystallinecellulose, hydroxyehthylcellulose, hydroxypropylcellulose, hydroxypropylmethylcellulose methylcellulose, guar gum, or corn starch. The chlorite salt of the biocide reacts with the acid to form germicidal chlorine dioxide and germicidal hypochlorous acid. Specifically, hydronium ions supplied from the acid react with chlorite salt to form the broad spectrum germicides chlorine dioxide and hypochlorous acid.

In one embodiment, where sodium chlorite is used as the chlorite salt, the reaction to form the germicides is as follows:

4 NaClO₂ + 2 H₃O⁺ -> 2 ClO₂⁻ + 2 HClO₂ + 4 Na⁺ + 2 H₂O

The acid constituency and the alkaline agent constituency of the biocide regulate the pH of the solution such that the biocide is stable when mixed for a period greater than two months. The pH of the solution is regulated to limit the supply of hydronium ions from the acid such that the chlorite salt reaction with the hydronium ions to form germicidal chlorine dioxide and hypochlorous acid is correspondingly regulated. The regulation of the pH helps achieve equilibrium between the hypochlorous acid and chlorine dioxide according to the following reaction:

HClO + 2 HClO₂⁻ < - > 2 ClO₂⁻ + H₂O + H⁺ + Cl⁻

The regulation of the pH of the solution through the acid and alkaline agent constituencies also regulates the formation of chlorate ions from hypochlorous acid. The production of chlorate ions (ClO₃⁻) causes the reduction in the shelf life of the chlorine dioxide and hypochlorous acid in solutions. Accordingly, stabilizing the amount of hypochlorous acid and keeping the pH from becoming too acidic significantly reduces the formation of the chlorate ions, further improving the stability of the prophylactic biocide disclosed herein. The production of the chlorate ions is regulated according to the following reaction:

ClO₂⁻ + Cl₂ + H₂O -> ClO₃⁻ + 2 Cl⁻ + 2 H⁺

The presence of urea further stabilizes the prophylactic biocide by complexing the chlorine dioxide and stabilizing the negative charge associated with this molecule. The non-stabilizing hydrogens in urea can be replaced with other alkyl groups thus causing a slight reduction in the energy of the complex and thus, theoretically increase the shelf-life of this invention further. The complexing/stabilization of the chlorine dioxide by urea is shown by the following anionic complex:

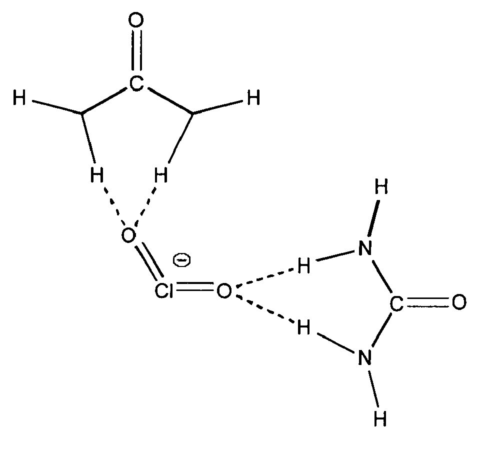

One embodiment of the stable, prophylactic biocide comprises: 0.10-20.0% by weight sodium chlorite, 0.10-3.0% by weight mixture of citric and lactic acids, 0.10-2.0% by weight sodium hydroxide, 0-0.65% by weight xanthan gum, 0-20.0% by weight linear alcoholic ethoxylate, 0-65% by weight glycerine, 0-2.0% by weight urea, 0-0.05% by weight FD&C Blue #1 dye, and the balance water.

In another embodiment, the stable, prophylactic biocide comprises 0.80% sodium chlorite, 1.1 % by weight lactic acid, 0.40% by weight sodium hydroxide, 0.50% by weight xanthan gum, 1.00% by weight linear alcohol ethoxylate, 15% by weight glycerine, 0.05% by weight FD&C Blue #1 dye, 0.80% by weight urea, and the remainder water.

All of the embodiments of the stable, prophylactic biocides described above have a germicidal shelf life greater than two months from the date of mixing. Thus, the very powerful, broad spectrum germicides of hypochlorous acid and chlorine dioxide, which have proven efficacy against a multitude of pathogenic microorganisms, may be manufactured and stored for a period of at least two months. This biocide is advantageous as it does not require a binary system for combination on site and, instead, one container may be used to hold the entire solution for use.

In accordance with the above, a method for producing a stable, prophylactic biocide for use with dairy animals, for example, as a teat dip, comprises combining 0.10-20.0% by weight of a chlorite salt, 0.10-3.0% by weight of an acid, 0.10-2.0% by weight of an alkaline agent, and the balance water in a container, and sealing the container. The container may be any suitable container, as are well known in the art. The acid and alkaline agent constituencies regulate the pH of the solution such that the formation of hydronium ions from the acid is correspondingly regulated to stabilize the solution for a period greater than two months from the date of sealing.

As described above, the combining of the chlorite salt and the acid forms germicidal chlorine dioxide and hypochlorous acid which are broad spectrum germicides that have proven efficacy against pathogenic microorganisms. The formation of chlorine dioxide and hypochlorous acid is regulated by the formation of the hydronium ion from the acid and the solution. The regulation of the pH of the solution also reduces the formation of chlorate ions derived from formed hypochlorous acid.

As noted above, the acid used in the mixture may be any acid, including those delineated above in the certain embodiments. Likewise, the chlorite salt may be any chlorite salt, and may include the chlorite salts listed above in the certain embodiments.

The step of combining in the method may further include binding up to 2.0% by weight urea, up to 65% by weight of an emollient, up to 0.65% of a rheology modifier, up to 20% by weight of a non-ionic surfactant, and up to 0.05% by weight of a dye. All of these additional constituencies used in the combining step may be of the types described above with respect to various embodiments of the stable, prophylactic biocide of the present application.

The average prophylactic biocide that utilizes both chlorine dioxide and hypochlorous acid is sold as a binary system and generates approximately 10,620 parts per million (ppm) of chlorine dioxide upon initial mixing. However, the chlorine dioxide levels drop to approximately 1,240 ppm after 24 hours. After only one week, the average mixed binary product contains less than 630 ppm chlorine dioxide, which is less than 10% of the original level.

In sharp contrast, the prophylactic biocide of the present application maintains high levels of chlorine dioxide for at least two months after initial mixing, with only a gradual decrease over 24 months and beyond.

In one experiment, a prophylactic biocide consisting essentially of 0.80% sodium chlorite, 1.1% by weight lactic acid, and 0.40% by weight sodium hydroxide demonstrated an initial chlorine dioxide level of approximately 10,600 ppm. After one month, the chlorine dioxide level remained high at approximately 10,370 ppm. After two months, the chlorine dioxide level remained over 10,000 ppm at approximately 10,120 ppm.

In another experiment, a prophylactic biocide consisting essentially of 0.80% by weight sodium chlorite, 1.1% by weight lactic acid, 0.40% by weight sodium hydroxide, and 1.00% by weight urea demonstrated an initial chlorine dioxide level of approximately 10,600 ppm. After one month, the chlorine dioxide level was measured at 10,450 ppm. Remarkably, after two months, the chlorine dioxide level remained very high at 10,180 ppm.

## Claims

1. A stable, prophylactic biocide consisting essentially of:
0.10-20.0% by weight of a chlorite salt;
0.10-3.00% by weight of an acid;
0.10-2.00% by weight of an alkaline agent;
and the balance water.

2. The biocide of claim 1, wherein the lower level of chlorite salt is 0.20 % by weight.

3. The biocide of claim 1 or 2, wherein the chlorite salt is selected from the group consisting of: sodium chlorite, magnesium chlorite, potassium chlorite and calcium chlorite, or a mixture thereof.

4. The biocide of any one of the preceding claims, wherein the acid is selected from the group consisting of: citric acid, phosphoric acid, lactic acid, acetic acid, acetylsalicylic acid, or a mixture thereof.

5. The biocide of any one of the preceding claims, wherein the alkaline agent is selected from the group consisting of: sodium hydroxide, potassium hydroxide, calcium hydroxide, sodium carbonate, potassium carbonate, or a mixture thereof.

6. The biocide of any one of the preceding claims, wherein the chlorite salt reacts with the acid to form germicidal chlorine dioxide and hypochlorous acid.

7. The biocide of any one of the preceding claims, wherein the acid constituency and the alkaline agent constituency regulate the pH of the solution such that the biocide is stable when mixed for a period greater than two months.

8. The biocide of claim 7, wherein the pH of the solution is regulated to limit supply of hydronium ions from the acid such that the chlorite salt reaction with the hydronium ions to form germicidal chlorine dioxide and hypochlorous acid is correspondingly regulated, and/or the pH of the solution is regulated such that the formation of chlorate ions from hypochlorous acid is reduced, preferably to a value between 6.5 and 6.9.

9. The biocide of any one of the preceding claims, wherein the biocide further includes up to 2.00% urea to enhance stability and promote wound healing.

10. The biocide according to claim 9, primarily for dairy animals, the biocide consisting essentially of:
0.10-20.0% by weight sodium chlorite;
0.10-3.00% by weight lactic and/or citric acid;
0.010-2.00% by weight urea;
0.10-2.00% by weight sodium hydroxide; and
the balance water, wherein the sodium chlorite reacts with hydronium ions supplied from the acid to form germicidal chlorine dioxide and hypochlorous acid, and
wherein the acid and sodium hydroxide constituencies regulate the pH of the solution such that the biocide is table when mixed for a period greater than two months.

11. The biocide of any one of the preceding claims, wherein the biocide further includes up to 0.05% of a dye.

12. The biocide of any one of the preceding claims, wherein the biocide further includes a perfume.

13. The biocide of any one of the preceding claims, wherein the biocide further includes up to 20.00% of a non-ionic surfactant,
wherein, preferably, the non-ionic surfactant is a chlorite-stable surfactant,
wherein, further preferably, the non-ionic surfactant is selected from the group consisting of: linear alcohol ethoxylate, nonylphenol ethoxylate, sodium xylene sulfates, and quaternary ammonium chlorites.

14. The biocide of any one of the preceding claims, wherein the biocide further includes up to 65% of an emollient,
wherein, preferably, the emollient is selected from the group consisting of: glycerin, sorbitol, propylene glycol, petrolatum, mineral oil, olive oil, coconut oil, lanolin and cocoa butter.

15. The biocide of any one of the preceding claims, wherein the biocide further includes up to 0.65% of a rheology modifier to achieve a preferred viscosity,
wherein, preferably, the rheology modifier is selected from the group consisting of: xanthan gum, carboxymethylcellulose, microcrystallinecellulose, hydroxyehthylcellulose, hydroxypropylcellulose, hydroxypropylmethylcellulose methylcellulose, guar gum, or corn starch.

16. The biocide of any one of the preceding claims, wherein the biocide is used as a dairy animal teat disinfectant.

17. A method of producing a stable, prophylactic biocide for dairy cattle, the method comprising:
combining 0.10-20.00% by weight of a chlorite salt, 0.10-3.00% by weight of an acid, 0.10-2.00% by weight of an alkaline agent, and the balance water in a container; and sealing the container; wherein the acid and alkaline agent constituencies regulate the pH of the solution such that the formation of hydronium ions form the acid is correspondingly regulated to stabilize the solution for a period greater than two months from the date of sealing,
wherein, preferably, the regulation of the pH of the solution reduces the formulation of chlorite ions derived from hypochlorous acid

18. The method of claim 17, wherein the combining of chlorate salt and the acid forms germicidal chlorine dioxide and hypochlorous acid,
wherein, preferably, the formation of chlorine dioxide and hypochlorous acid is regulated by the formation of the hydronium ion.

19. The method of claim 17 or 18, wherein the acid is selected from the group consisting of: citric acid, phosphoric acid, lactic acid and acetic acid, or a mixture thereof.

20. The method of claim 17, 18 or 19, wherein the chlorite salt is selected from the group consisting of: sodium chlorite, magnesium chlorite, potassium chlorite and calcium chlorite.

21. The method of any one of the claims 17 to 20, wherein the step of combining further includes combining up to 2.0% urea, up to 65% of an emollient, up to 0.65% of a rheology modifier, up to 20.0% of a non-ionic surfactant, and up to 0.05% of a dye.
